Europäisches Patentamt

European Patent Office

Office européen des brevets

(19)

(11) Publication number: **0 273 900**
**B1**

# EUROPEAN PATENT SPECIFICATION

(45) Date of publication of the patent specification:
27.06.90

(21) Application number: **87870211.7**

(22) Date of filing: **30.12.87**

(51) Int. Cl.⁵: **C10G 45/62,** C07C 7/167,
C10G 65/04, B01J 23/44,
B01J 23/72

(54) Improved process for the selective hydrogenation of acetylenes.

(30) Priority: **30.12.86 GB 8631018**

(43) Date of publication of application:
**06.07.88 Bulletin 88/27**

(45) Publication of the grant of the patent:
**27.06.90 Bulletin 90/26**

(84) Designated Contracting States:
**AT BE CH DE ES FR GB IT LI NL SE**

(56) References cited:
**EP-A- 0 139 051**
**DE-A- 2 913 209**

(73) Proprietor: FINA RESEARCH S.A., Zone Industrielle C,
B-6520 Seneffe(BE)

(72) Inventor: **Debras, Guy L.G., 12, rue du Champ de la
Chapelle, B-6410 Les-Bons-Villers(BE)**
Inventor: **De Clippeleir, Georges E.M.J., 5, Petrus
Huysegomstraat, B-1600 Sint-Pieters-Leeuw(BE)**
Inventor: **Cahen, Raymond M., 21, Avenue Commandant
Lothaire, B-1040 Bruxelles(BE)**
Inventor: **Grootjans, Jacques F., 39, Neerijsesteenweg,
B-3061 Leefdael(BE)**

(74) Representative: **Leyder, Francis, c/o Fina Research S.A.
Zone Industrielle C, B-6520 Feluy(BE)**

## Description

The present invention relates to an improved process for removing alkynes from liquid hydrocarbon streams with a minimum loss of conjugated dienes present therein. More specifically, the invention relates to the selective hydrogenation of the alkynes present in the 1,3-butadiene-rich $C_4$ cuts from the stream cracking units and mainly used for the production of synthetic rubber.

The polymerization of 1,3-butadiene to produce synthetic rubber is an important industrial process, some 10 million tons being produced annually. The typical feedstocks used contain a major proportion of 1,3-butadiene and butenes but they also contain significant amounts of alkynes (also called acetylenes), mainly vinylacetylene. As acetylenes act as a catalyst poison in the polymerization, they should be removed as completely as possible. Accordingly, it is usual to selectively hydrogenate the acetylenic compounds while trying to avoid or to limit losses in 1,3-butadiene.

The selectivity requirements for the process are high, since all other reactions should be avoided or prevented as much as possible. Said reactions obviously include the hydrogenation of 1,3-butadiene and butenes, but also polymerization reactions which reduce the catalyst life. Regenerations of the catalyst are possible, but the frequence thereof is economically important, while they induce catalyst modifications and eventually mechanical breakdown of the catalyst pellets which results in higher pressure drops across the bed.

It has long been known to selectively hydrogenate at high temperature in vapour phase over a copper-nickel catalyst on a $SiO_2/Al_2O_3$ support.

However, such processes are increasingly being abandoned because the catalyst has to be replaced or regenerated frequently, while the 1,3-butadiene loss and the residual acetylenes concentration are presently considered as being too important.

It is also known to use copper-based catalysts for the selective hydrogenation of alkynes in liquid hydrocarbon streams.

U.S. Patent 4,493,906 discloses a catalyst for the removal of acetylenes from liquid hydrocarbon streams, said catalyst consisting essentially of finely divided Cu metal dispersed on a well-defined gamma alumina (which may contain up to 35 wt % of alpha alumina). The gamma alumina used has a surface area of 68-350 $m^2/g$; 40-98% of the pores have a pore diameter of 4-12 nm, and 2-25% have a pore diameter between 100 and 1000 nm. The support is of high purity, with silicon less than 0.15 wt % as $SiO_2$ and Na < 0.15 wt % as $Na_2O$. The catalyst of U.S. Patent 4,493,906 is claimed to leave 0 ppm acetylenics when used at about 68°C and with a LHSV (liquid hourly space velocity) lower than 1. However, the corresponding cycle life is of only 5 1/2 days; after 6 days, about 100 ppm acetylenes are detected in the effluent. Obviously, at higher values of LHSV, either the cycle life would be shorter, or the acetylenes removal would not be complete.

Another type of catalysts is palladium-based. Palladium is, among the metals of Group VIII, the most active and selective metal for the hydrogenation of acetylenics. However, it is known in the art that two types of operating troubles are encountered :
- 1,3-butadiene losses are observed even at moderate conversion of alkynes; and
- Palladium losses often reduce the catalyst life;
as clearly disclosed inHydrocarbon Processing, March 1985, p. 52.

As time goes by, the severity of the steam cracking increases, and the $C_4$ raw cuts thus contain increasing concentrations of alkynes, up to 1 wt % or even higher. On the other hand, the requirements for the acetylenics concentration in the effluent from the selective hydrogenation are becoming more and more severe. There is accordingly a need in the art for an improved process for removing alkynes from liquid hydrocarbon streams with a minimum loss of conjugated dienes present therein.

The process of the present invention for the selective hydrogenation of the alkynes present in 1,3-butadiene-rich $C_4$ cuts comprises the steps of :

(i) providing a 1,3-butadiene-rich $C_4$ cut;
(ii) passing said cut over a palladium-based catalyst bed in the presence of hydrogen;
(iii) passing the liquid effluent from step (ii) over a copper-based catalyst bed in the presence of hydrogen;
(iv) separating the residual hydrogen from the remainder of the effluent from step (iii); and
(v) recovering a 1,3-butadiene-rich feedstock.

Palladium-based catalysts which may be used in the process of invention are well known in the art. A particularly preferred catalyst consists of active palladium metal deposited on a high purity alumina support.

The amount of palladium in the catalyst which includes a high purity alumina support is preferably 0.1 to 0.35 wt %, more preferably about 0.2 wt %. Advantageously, the alumina is of high purity and the concentration of heavy metals other than Pd is less than 0.05 wt %.

The surface area of the catalyst is preferably 50-110 $m^2/g$, more preferably 65-95 $m^2/g$. The volume of the pores is preferably 0.5 - 0.6 $cm^3/g$. The catalyst is preferably in the form of spheres of 2-4 mm size.

It has already been said that the acidity of the alumina influences the undesired oligomerization reactions, and that gamma-alumina should therefore be preferred to a conventional eta-alumina. However, this is not required by the process of the invention, because this concerns only the long-term stability, not the activity of the fresh or regenerated catalyst. Other types of alumina may also be used, such as Q-type alumina, which has been disclosed in Japanese Patent Application JP-58017835.

So-called stabilized or promoted palladium-based catalysts are well known, as e.g. the palladium-gold supported catalysts disclosed in European Patent EP-89,252. However, the activity of these catalysts is usually lower than that of palladium-based catalysts. Although not wishing to be bound by a theory, this could be explained by a less homogeneous dispersion of the metals on the support, because it is almost impossible to obtain a controlled supported bimetallic catalyst at the low loading levels used for industrial precious metals catalysts. Indeed, the carrier must be suitable for appropriate interaction between the metals, and a well dispersed bimetallic species must be obtained and maintained. The use of stabilized or promoted catalysts in the process of the invention is optional and will therefore depend on the activity and long-term stability requirements.

The activation, start-up and regeneration procedures of the palladium-based catalysts are known in the art. The activation consists of (i) purging the oxygen using nitrogen, and (ii) passing hydrogen under atmospheric pressure while gradually heating up to a level of about 90°C, then cooling. The start-up procedure consists of slowly increasing the hydrogen pressure, then the feed and hydrogen flow rates, and finally the temperature. The regeneration procedure consists of passing steam under atmospheric pressure while gradually increasing the temperature to about 400°C, then continuing to pass steam under atmospheric pressure at a temperature of about 400°C during about two hours thereafter, and finally gradually adding up to several mol percent air to said steam. During the regeneration procedure, the catalyst temperature should not exceed about 500°C. The regeneration is complete when the $CO_2$ content at the exit is sufficiently low.

The prior art and the catalysts manufacturers recommend the following typical process conditions for the selective hydrogenation of vinyl and ethyl acetylene in a liquid 1,3-butadiene-rich $C_4$ cut, using palladium-based catalysts :
- temperature : 15-20°C (inlet)
- pressure : 0.5 MPa (5 bar)
- LHSV : 30 l/l.h
- $H_2$/alkynes molar ratio : 2:1

The typical results obtained with these conditions are :
- feed : 1,3-butadiene 50 vol. %
ethyl acetylene 0.2 vol. %
vinyl acetylene 1.2 vol. %
balance = butenes
- purified effluent : 500 ppm total alkynes
3% butadiene loss
- cycle life : 8-10 months

Copper-based catalysts which may be used in the process of the invention are also well known in the art. They preferably comprise 3-13 wt % of finely divided Cu dispersed on high purity aluminium oxide support. U.S. Patent 4,493,906 cited hereabove discloses copper-based catalysts and, in particular, catalysts which may be used in the present invention.

Typical process conditions for the selective hydrogenation of vinyl and ethyl acetylenes in a liquid 1,3-butadiene-rich $C_4$ cut, using copper-based catalysts, are as follows :
- activation by overnight hydrogenation at 300-350°C,
- inlet temperature 68°C,
- pressure : 2-2.5 MPa (20-25 bars),
- hydrogen : alkynes molar ratio : 3:1,
- LHSV : 0.67

The typical results obtained are :
- feed composition : 61 wt % 1,3-butadiene
8716 ppm acetylenes
- purified effluent : 60.5 wt % 1,3-butadiene
0 ppm acetylenes
- cycle length : 5 1/2 days

So-called stabilized or promoted copper-based catalysts are well-known, as e.g. the catalysts disclosed in European Patent Application EP-139,051. However, the performances of such catalysts are much comparable with those of the plain copper-based catalysts, and both may be used in the process of the invention.

The Applicants have unexpectedly found that the selective hydrogenation process is improved by combining two successive reactors, the first of which contains a palladium-based catalyst and the second containing a copper-based catalyst. It has been found that such combination allows the best selectivity during very long run times to be obtained. Accordingly, the improved process gives an effluent that meets the requirements for the residual acetylenes concentration while keeping the 1,3-butadiene losses to a minimum, said process requiring both lesser amounts of catalysts and less frequent regenerations than those of the prior art.

The optimal conditions for the hydrogenation over a copper-based catalyst have been found to be as follows. With a 100% $H_2$ flow the total pressure should preferably be from 0.4 to 0.9 MPa, most preferably from 0.6 MPa to 0.8 MPa. Thus, if refinery hydrogen is used in the process of the invention, said refinery hydrogen usually containing about 75% hydrogen and about 25% methane, the total pressure should be slightly higher. The inlet temperature of the feed should be sufficiently low to keep the feed at least partially in the liquid state, preferably at a temperature of from 45 to 70°C.

The LHSV should be less than about 6 l per l of copper-based catalyst and per hour, preferably about 5.5 l/l.hour. In this step, the hydrogen/acetylenes molar ratio should be at least 5 : 1, but there is no upper limit for this ratio since the 1,3-butadiene loss does not exceed 0.5 wt % of its initial concentration.

However, these conditions do not allow the residual alkynes specifications to be met when the feed contains more than 300-400 ppmw of alkynes.

At the laboratory scale, it is possible to reduce the LHSV so that the feed contains more than 300-400 ppmw of alkynes. However, when the feed contains up to 1 wt % or higher amounts of alkynes, the LHSV that would be required would be so low that an industrial application of the purification of an 1,3-butadiene-rich feed for a rubber plant would require such large vessels that the process would be impracticable. Also, the pressure has to be considerably increased which is obviously undesirable.

It was unexpectedly found that an improved process was obtained by previously reducing the initial alkynes concentration to about 300-400 ppmw by means of a selective hydrogenation of the feed over a palladium-based catalyst. The typical operation conditions for this step are usually the following :
- total pressure : from 0.4 to 0.9 MPa, preferably of from 0.6 to 0.8 MPa;
- hydrogen/alkynes molar ratio : from 2:1 to 20:1, preferably of from 4:1 to 10:1, most preferably of about 6:1;
- inlet temperature : adjusted with respect to the total pressure in order to maintain the feed at least partially in the liquid state.

Using these typical conditions, the LHSV of the feed should be adjusted in such a way that the alkynes concentration after passing over the palladium-based catalyst is of about 300-400 ppmw. This is usually obtained by using a LHSV value, expressed in l/l.h, such that the product of the LHSV by the initial alkynes concentration, expressed in wt %, is about 15.

In the process of the invention, each reactor may be either an isothermal reactor or an adiabatic reactor. The hydrogen may be injected with the feed, although it has been found highly desirable to inject the hydrogen partially with the feed and partially in the course of the process, e.g. at one or several places about half way along the first catalyst bed and at the second reactor inlet. According to an embodiment of the invention, up to 30%, more preferably about 15%, of the total hydrogen flow is injected at one or several places, up to the second reactor inlet.

The Applicants have unexpectedly found that the known palladium-based catalysts are more selective when used in trickle mode than in homogeneous liquid phase. The term "increased selectivity", as used herein, means that, for a given feed, less 1,3-butadiene is lost for a given level of alkynes hydrogenation. The term "trickle mode", as used herein, is defined as the operation under such conditions of temperature and pressure that cause the feed to pass as a mixed gaseous-liquid phase over the catalyst. The hydrogenation being an exothermal reaction, it is usually more convenient to provide the feed in the liquid state, under condtions very near to the gaseous-liquid equilibrium. If an adiabatic reactor is used as first reactor, in trickle mode, the heat released by the hydrogenation reaction is compensated by the vaporization of part of the liquid phase. It is thus highly desirable in the case of an adiabatic reactor used in trickle mode to have at the inlet enough feedstock in the liquid phase in order to absorb said heat release, and further to inject part of the feed in the liquid form along the axis of the reactor. According to an embodiment of the invention, a sufficient proportion, preferably up to about 20%, and more preferably 5-10% of the feed is injected in liquid form, at one or several places, preferably about half way, of the catalyst bed in an adiabatic reactor, when the reactor is used in trickle mode. Although not wishing to be bound by a theory, the Applicant believes that these injections possibly serve to maintain the trickle mode conditions constant throughout the adiabatic reactor.

Considering that the gaseous phase in the trickle mode is produced partially by the vaporization of the feed, the trickle mode is usually operated in co-current mode. Although it is possible to operate in upflow mode, the Applicant has found that it is highly preferable to operate in down-flow mode.

When operating in trickle mode, the pressure in the reactor is adjusted with respect to the temperature, in order to maintain the desired trickle mode operation. Within the preferred ranges, higher values of the pressure and temperature tend to impart a higher activity to the catalyst.

The feeds that may be used in the process of the invention usually comprise a mixture of normally gaseous hydrocarbons, i.e.
- 1,3-butadiene 30-55 %, typically 40-50%,
- 1,2-butadiene up to 2%, typically 0.2%,
- alkynes mainly up to 5%, typically up to 1.5%;
(ethyl and vinyl acetylene)
- $C_3$ hydrocarbons and heavies traces
- butanes up to 10%, typically up to 5%,

- butenesbalance

These feeds are usually obtained from the steam cracking unit. However, other feeds or feeds obtained from other sources may also be contemplated, without departing from the scope of the invention.

The invention will now be illustrated by means of the following examples which are not meant to be limitative.

Example 1

a. Preparation of the palladium-based catalyst

The alumina support selected was under the form of spheres having a diameter of 2-4 mm and a bulk density of 0.72 g/cm³.

The support was contacted with a solution of palladium acetylacetonate in benzene. The weight ratio support : solution was of 10 : 16. The Pd weight concentration in the solution was of 1350 ppmw before contacting the solution with the support, and of 100 ppmw after 8 hours of impregnation.

The impregnated support was filtered and dried at 120°C during 6 hours under an air flow. It has been heated at 300°C in a tubular oven, first during 2 hours under an air flow, then, after a nitrogen flush, during a further 2 hours under an hydrogen flow.

After cooling, the catalyst contained 0.2 wt % of palladium.

b. Activation and start-up of the palladium-based catalyst

The catalyst was purged during one hour with nitrogen at a space velocity of 333 l/l.h. Hydrogen under atmospheric pressure was then passed over the catalyst at a space velocity of 200 l/l.h; the catalyst was then heated at 66°C during 0.5 hours, then at 93°C during 2 hours, and finally cooled to 20°C. The hydrogen used was refinery hydrogen, consisting of a mixture of about 75% hydrogen and about 25% methane !

The hydrogen flow was then increased to 333 l/l.h at a temperature of 26°C for 35 minutes. The hydrogen pressure was then slowly increased from atmospheric to 0.61 MPa (6.2 kg/cm²) and maintained for 45 minutes. The feed and hydrogen flow rates were then increased to one fourth of nominal, maintained for 50 minutes, increased to half of the nominal values, maintained for 15 minutes, and finally increased to nominal values while raising the temperature to 57°C at a heating rate of 10°C/h.

c. Preparation of the copper-based catalyst

A special grade of gamma-alumina was prepared by decomposing triethylaluminium to alpha aluminium monohydrate, then calcining the alpha aluminium monohydrate to gamma alumina.

The resulting powder was extruded into pellets of about 1.5 mm diameter and about 6 mm length. The following properties were determined :
% $Na_2O$ 0.015
% $Fe_2O_3$ 0.006
Surface area (m²/g) 240
Pore volume (cm³/g) 0.56
Bulk density (g/cm³) 0.69
Pores < 7.5 nm 75 %
Pores < 10 nm 82 %

The extrudates were impregnated in a weight ratio of 20:9 with a solution of 77.8 wt % 2 $Cu(NO_3)_2.5$ $H_2O$ in water. When all the solution had been absorbed, the support was dried overnight at about 110°C, then calcined at 400°C for about 6 hours.

d. Activation and start-up of the copper catalyst

The catalyst was purged during 4 hours under atmospheric pressure and at a temperature of 150°C with nitrogen at a space velocity of 50 l/l.h To the nitrogen flow was then added during 24 hours a flow of hydrogen at a space velocity of 5 l/l h, all other conditions being identical. The temperature was then increased to 280°C during a further 24 hours, after what the hydrogen flow was interrupted and the catalyst was allowed to cool under the nitrogen flow.

e. Alkynes selective hydrogenation

The feed contained :
- 45.90 wt % of 1,3-butadiene
- 4890 ppmw of vinylacetylene (VAC)
- 1110 ppmw of ethylacetylene (EAC)

5

- 1010 ppmw of methyl acetylene (MAC)
about 50% of butenes, about 4% of butanes, and traces of other hydrocarbons.

The hydrogenation was carried out in two serial reactors. The first reactor was an adiabatic reactor, containing the palladium-based catalyst, operated in downflow mode. Refinery hydrogen containing 74% hydrogen was used. The gauge pressure was of 0.82 MPa (8.2 bar); the inlet temperature was adjusted to 64°C, so that this step was carried out in trickle-mode. The feed was injected with a total space velocity corresponding to 24.7 litres of liquid feed per litre of catalyst and per hour. However, 8.1% of the feed was injected at a side inlet about half way of the catalyst bed. Hydrogen was injected with the feed, in an hydrogen : feed molar ratio of 0.054 at the main inlet and of 0.048 at the side inlet. After the first reactor, a sample was taken. The 1,3-butadiene loss was of 3.1 wt % of the initial amount, and the sample contained 27 ppmw of VAC and 362 ppmw of EAC.

The second reactor was an adiabatic reactor containing the copper-based catalyst, operated in upflow mode. The inlet temperature was of 58°C and the gauge pressure was of 0.8 MPa. The feed was passed with a liquid hourly space velocity (LHSV) of 6.0 l/l.h. Hydrogen was injected with the feed, in sufficient amount to reach an hydrogen : alkynes molar ratio of 150. The effluent contained less than 5 ppmw (detection limit) of any of VAC, EAC or MAC. The 1,3-butadiene loss in the second reaction was of 0.3 wt % of the initial amount, and the total 1,3-butadiene loss was thus of 3.4 wt % of said initial amount.

Example 2

The experiment of Example 1 was repeated with a similar feed containing 46.36 wt % of 1,3-butadiene, 7880 ppmw of VAC and 1690 ppmw of EAC. All experimental conditions were identical, except the following :

a) first reactor
inlet temperature: 66°C
feed injected at the main inlet: 100%
LHSV: 14.7 l/l.h
$H_2$ : feed molar ratio: 0.047
    b) second reactor
inlet temperature: 56°C
LHSV: 6.1 l/l.h
$H_2$ : alkynes molar ratio: 120

After the first reactor, the 1,3-butadiene loss was of 2.6 wt % of the initial amount, and the effluent contained < 5 ppmw of VAC and 286 ppmw of EAC.

After the second reactor, the corresponding figures were 0.3 wt %, < 5 ppmw and < 5 ppmw. The total 1,3-butadiene loss was thus of 2.9 wt % of the initial amount.

Comparative example A

A palladium-based catalyst was prepared according to the procedure described in Example 1, and its activation and start-up were likewise carried out.

The feed contained :
46.99 wt % of butadiene
7140 ppmw of VAC
1680 ppmw of EAC
47.89 wt % of butenes
3.74 wt % of butanes
the balance consisting of other hydrocarbons.

The hydrogenation was carried out in an isothermal reactor, containing the palladium-based catalyst, operated in downflow mode. The pressure was of 0.5 MPa (5 bar), the temperature was of 20°C. The liquid hourly space velocity of the feed was of 30 l/l.h. Pure hydrogen was injected with the feed, in an hydrogen : feed molar ratio of 0.04.

The 1,3-butadiene loss was of 3 wt % of the initial amount, and the effluent contained more than 500 ppmw of each of VAC and EAC.

This example shows that the conditions usually recommended in the prior art do not allow sufficiently low residual acetylenes concentrations to be obtained.

Comparative example B

The experiment described in comparative example A was repeated with different hydrogenation conditions.

The hydrogenation was carried out in an adiabatic reactor, operated in downflow mode using pure hydrogen. The inlet temperature was of 50.5 °C. The feed LHSV was of 14.2, and the hydrogen : feed molar ratio was of 1 : 20.
The results are shown in Table 1.

Table 1

|  | Feed | Effluent A | Effluent B | Effluent C |  |
|---|---|---|---|---|---|
| Pressure |  | 0.61 | 0.69 | 0.78 | MPa |
| Composition |  |  |  |  |  |
| 1,3-butadiene | 46.99 | 44.23 | 43.98 | 43.71 | wt % |
| VAC | 7140 | 109 | 258 | 487 | ppmw |
| EAC | 1680 | 282 | 375 | 455 | ppmw |
| butanes | 3.74 | 3.75 | 3.77 | 3.79 | wt % |
| butenes | 47.89 | 51.60 | 51.78 | 52.02 | wt % |
| other hydroc. | balance | balance | balance | balance |  |

This example shows that, despite considerable 1,3-butadiene losses, the effluent still contains important amounts of VAC and EAC.

Comparative Example C

A palladium-based catalyst was prepared according to the procedure described in Example 1, and its activation and start-up were likewise carried out.
The feed contained :
41.04 wt % of 1,3-butadiene,
7280 ppmw of VAC,
1640 ppmw of EAC,
45,91 wt % of butenes,
8.71 wt % of butanes,
the balance consisting of other hydrocarbons.
The hydrogenation was carried out in an adiabatic reactor, containing the palladium-based catalyst, operated in down-flow mode using refinery hydrogen. The pressure was of 0.8 MPa (8 bars); the temperature was adjusted to 66°C so that this step was carried out in trickle mode. The feed was injected with a total space velocity corresponding to 7.2 litres of liquid feed per litre of catalyst and per hour. However, 10% of the feed were injected at a side inlet about half way of the catalyst bed. Hydrogen was injected with the feed, in an hydrogen : feed molar ratio of 0.081 at the main inlet and of 0.15 at the side inlet.
The 1,3-butadiene loss was of 8.0 wt % of the initial amount, and the effluent contained less than 5 ppmw (detection limit) of either VAC or EAC.
This example shows that, even with the improvement over the prior art consisting of working in trickle mode, the selectivity of palladium-based catalyst is not sufficient when used alone. Indeed, the total hydrogenation of VAC and EAC could only be obtained with greater 1,3-butadiene losses than with the process of the invention.

Comparative Example D

A copper-based catalyst was prepared according to the procedure described in Example 1, and its activation and start-up were likewise carried out.
The feed contained 46.18 wt % of 1,3-butadiene, 7679 ppmw of VAC and 1825 ppmw of EAC, the balance mainly consisting of butenes.
The hydrogenation was carried out in an adiabatic reactor, containing the copper-based catalyst, operated in upflow mode. The inlet temperature was of 51°C, and the pressure was of 1.7 MPa (17 bars). The liquid space velocity of the feed was of 1 l/l.h. Refinery hydrogen was injected with the feed, in an hydrogen : feed molar ratio of 0.024.
The 1,3-butadiene loss was of 3.0 wt % of the initial amount, and the effluent contained less than 5 ppmw (detection limit) of either VAC or EAC.
This example shows that, although a good selectivity may be obtained by using a copper-based catalyst, the process of the invention allows the same results at lower pressure and higher space velocity to be obtained.
Indeed, if either lower pressures or higher space velocities are used in an experiment similar to this comparative example D, residual alkynes may be detected in the effluent.

Comparative Example E

A palladium-based catalyst and a copper-based catalyst were prepared according to the procedures described in Example 1, and their activation and start-up were likewise carried out.

The feed contained 44.34 wt % of 1,3-butadiene, 7579 ppmw of VAC and 1865 ppmw of EAC, 49,75 wt % of butenes, 3.95 wt % of butanes, the balance consisting of other hydrocarbons.

The hydrogenation was carried out in two serial reactors using refinery hydrogen. The first reactor was an adiabatic reactor, containing the copper-based catalyst, operated in downflow mode. The pressure was of 0.85 MPa (8.5 bars) and the inlet temperature was of 52.4°C. The feed was injected with a liquid space velocity of 1.0 l/l.h, together with hydrogen in an hydrogen : feed molar ratio of 0.023. After the first reactor, the effluent contained 940 ppmw of VAC and 1790 ppmw of EAC, and the 1,3-butadiene concentration was slightly increased to 44.63 wt %.

The second reactor was an adiabatic reactor, containing the palladium-based catalyst, operated in downflow mode. The pressure was of 0.8 MPa (8 bars), and the inlet temperature was adjusted to 65°C so that this step was carried out in trickle mode. The feed was injected with a total space velocity corresponding to 7.5 litres of liquid feed per litre of catalyst and per hour. Hydrogen was injected with the feed, in order to reach an hydrogen : alkynes molar ratio of 5. The effluent contained less than 5 ppmw (detection limit) of either VAC or EAC. The 1,3-butadiene loss in the second reactor was of 8.25 wt % of the initial amount, and the total 1,3-butadiene loss was thus of 7.6 wt % of said initial amount.

This example shows that any other combination of copper- and palladium-based catalysts does not give the improvement provided by the process of the invention. Indeed, the total hydrogenation of VAC and EAC could only be reached with (i) greater 1,3-butadiene losses and (ii) lower space velocities than with the process of the invention.

The preferred embodiments of the present invention provide an improved process for removing alkynes from hydrocarbon streams, and also an improved process for removing alkynes from hydrocarbon streams with a minimum loss of the conjugated dienes present therein.

The preferred embodiments of the present invention also provide a process for removing alkynes from hydrocarbon streams down to less than 30 ppm.

The preferred embodiments of the present invention further provide a process for removing alkynes from hydrocarbon streams, wherein the catalyst life is greatly increased.

The preferred embodiments of the present invention still further provide a process for the selective hydrogenation of the alkynes present in the 1,3-butadiene-rich $C_4$ cuts from the steam cracking units and mainly used for the production of synthetic rubber.

## Claims

1. Process for the selective hydrogenation of alkynes present in 1,3-butadiene-rich $C_4$ cuts, the process comprising the steps of :
   (i) providing a 1,3-butadiene-rich $C_4$ cut;
   (ii) passing said cut at least partially in the liquid state over a first, palladium-based, catalyst in the presence of hydrogen;
   (iii) passing the effluent from step (ii) at least partially in the liquid state over a second, copper-based, catalyst in the presence of hydrogen;
   (iv) separating the residual hydrogen from the remainder of the effluent from step (iii); and
   (v) recovering a 1,3-butadiene-rich feedstock.

2. Process according to Claim 1, wherein the total pressure is from 0.4 MPa to 0.9 MPa when using a 100% hydrogen flow.

3. Process according to Claim 2, wherein the total pressure is from 0.6 MPa to 0.8 MPa.

4. Process according to any one of Claims 1 to 3, wherein the hydrogen:alkynes molar ratio is from 2:1 to 20:1.

5. Process according to Claim 4, wherein the hydrogen:alkynes molar ratio is from 4:1 to 10:1.

6. Process according to Claim 5, wherein the hydrogen:alkynes molar rato is about 6:1.

7. Process according to any foregoing claim, wherein up to 30% of the total hydrogen flow is injected at one or several places along the first catalyst bed, up to the second reactor inlet.

8. Process according to any foregoing claim, wherein the $C_4$ cut is passed in trickle mode over the first catalyst.

9. Process according to any foregoing claim, wherein step (ii) is carried out in an adiabatic reactor.

10. Process according to any foregoing claim, wherein a sufficient proportion of the feed is injected in liquid form at one or several places along the first catalyst bed.

11. Process according to Claim 10, wherein up to about 20% of the feed is injected in liquid form, at one or several places about half way along the first catalyst bed.

12. Process according to any foregoing claim, wherein step (ii) is operated in downflow mode.

13. Process according to any foregoing claim, wherein the first catalyst contains 0.1 to 0.35 wt % of active palladium metal deposited on high purity alumina.

14. Process according to Claim 13, wherein the amount of active palladium metal in the first catalyst is about 0.2 wt %.

15. Process according to any foregoing claim, wherein the first catalyst is stablized by the use of a bimetallic catalyst.

16. Process according to Claim 15, wherein the first catalyst is made of platinum-gold alloy deposited on high purity alumina.

17. Process according to any foregoing claim, wherein the LHSV of the feed in step (ii) is adjusted in such way that the alkynes concentration after passing over the palladium-based catalyst is from 300 to 400 ppmw.

18. Process according to any one of Claims 1 to 16, wherein the feed is passed in step (ii) at a LHSV, expressed in l/l.h, such that its product by the initial alkynes concentration, expressed in wt %, is about 15.

19. Process according to any foregoing claim, wherein the inlet temperature of the feed in step (iii) is from 45 to 70°C.

20. Process according to any foregoing claim, wherein the LHSV of the feed in step (iii) is less than about 6 l/l.h.

21. Process according to Claim 20, wherein the LHSV of the feed in step (iii) is about 5.5 l/l.h.

22. Process according to any foregoing claim, wherein the hydrogen:alkynes molar ratio in step (iii) is greater than about 1:5.

23. Process according to any foregoing claim, wherein the second catalyst comprises from 3 to 13 wt % of finely divided copper dispersed on a high purity aluminium oxide support.

24. Process according to any foregoing claim, wherein the aluminium oxide support of the second catalyst is prepared by decomposing triethylaluminium to alpha aluminium monohydrate which is then calcined to gamma alumina.

## Patentansprüche

1. Verfahren zur selektiven Hydrierung von Alkinen in $C_4$-Fraktionen mit einem hohen Gehalt an 1,3-Butadien, das folgende Stufen umfaßt:

(i) Bereitstellen einer $C_4$-Fraktion mit einem hohen Gehalt an 1,3-Butadien;

(ii) Passage der Fraktion zumindest teilweise im flüssigen Zustand über einen ersten Katalysator auf der Basis von Palladium in Gegenwart von Wasserstoff;

(iii) Passage des aus Stufe (ii) ausströmenden zumindest teilweise in flüssigem Zustand vorliegenden Produkts über einen zweiten auf der Basis von Kupfer in Gegenwart von Wasserstoff;

(iv) Abtrennen des restlichen Wasserstoffs vom Rest des aus Stufe (iii) ausströmenden Produkts; und

(v) Gewinnen eines Ausgangsmaterials mit einem hohen Gehalt an 1,3-Butadien.

2. Verfahren nach Anspruch 1, wobei der Gesamtdruck bei Verwendung eines 100%-Wasserstoffstroms 0,4 MPa bis 0,9 MPa beträgt.

3. Verfahren nach Anspruch 2, wobei der Gesamtdruck 0,6 MPa bis 0,8 MPa beträgt.

4. Verfahren nach einem der Ansprüche 1 bis 3, wobei das Molverhältnis Wasserstoff:Alkine 2:1 bis 20:1 beträgt.

5. Verfahren nach Anspruch 4, wobei das Molverhältnis Wasserstoff:Alkine 4:1 bis 10:1 beträgt.

6. Verfahren nach Anspruch 5, wobei das Molverhältnis Wasserstoff:Alkine etwa 6:1 beträgt.

7. Verfahren nach einem der vorstehenden Ansprüche, wobei bis zu 30% des gesamten Wasserstoffstroms an einer oder mehreren Stellen entlang des ersten Katalysatorbettes bis hin zum zweiten Reaktoreinlaß injiziert werden.

8. Verfahren nach einem der vorstehenden Ansprüche, wobei die $C_4$-Fraktion in einem Rieselverfahren über den ersten Katalysator geleitet wird.

9. Verfahren nach einem der vorstehenden Ansprüche, wobei die Stufe (ii) in einem adiabatischen Reaktor durchgeführt wird.

10. Verfahren nach einem der vorstehenden Ansprüche, wobei ein ausreichender Anteil des Ausgangsmaterials in flüssiger Form an einen oder mehreren Stellen entlang des ersten Katalysatorbettes injiziert wird.

11. Verfahren nach Anspruch 10, wobei bis zu etwa 20% des Ausgangsmaterials in flüssiger Form ab einer oder mehreren Stellen auf etwa der halben Strecke des ersten Katalysatorbettes injiziert werden.

12. Verfahren nach einem der vorstehenden Ansprüche, wobei die Stufe (ii) in Strömungsrichtung nach unten durchgeführt wird.

13. Verfahren nach einem der vorstehenden Ansprüche, wobei der erste Katalysator 0,1 bis 0,35 Gew.-% aktives Palladiummetall, das auf Aluminiumoxid von hoher Reinheit abgeschieden ist, enthält.

14. Verfahren nach Anspruch 13, wobei die Menge an aktivem Palladiummetall im ersten Katalysator etwa 0,2 Gew.-% beträgt.

15. Verfahren nach einem der vorstehenden Ansprüche, wobei der erste Katalysator unter Verwendung eines bimetallischen Katalysators stabilisiert ist.

16. Verfahren nach Anspruch 15, wobei der erste Katalysator aus einer Platin-Gold-Legierung, das auf Aluminiumoxid von hoher Reinheit abgeschieden ist, hergestellt ist.

17. Verfahren nach einem der vorstehenden Ansprüche, wobei der LHSV-Wert des Ausgangsmaterials in Stufe (ii) so eingestellt wird, daß die Konzentration der Alkine nach Durchlaufen des Katalysators auf Palladiumbasis 300 bis 400 ppm (Gew.) beträgt.

18. Verfahren nach einem der Ansprüche 1 bis 16, wobei das Ausgangsmaterial in Stufe (ii) mit einem LHSV-Wert, angegeben in Liter/Liter.h, durchgeleitet wird, so daß das Produkt aus dem LHSV-Wert und der ursprünglichen Konzentration an Alkinen, angegeben in Gew.-%, etwa 15 beträgt.

19. Verfahren nach einem der vorstehenden Ansprüche, wobei die Einlaßtemperatur des Ausgangsmaterials in Stufe (iii) 45 bis 70% beträgt.

20. Verfahren nach einem der vorstehenden Ansprüche, wobei der LHSV-Wert des Ausgangsmaterials in Stufe (iii) weniger als etwa 6 Liter/Liter.h beträgt.

21. Verfahren nach Anspruch 20, wobei der LHSV-Wert des Ausgangsmaterials in Stufe (iii) etwa 5,5 Liter/Liter.h beträgt.

22. Verfahren nach einem der vorstehenden Ansprüche, wobei das Molverhältnis Wasserstoff:Alkine in Stufe (iii) mehr als etwa 1:5 beträgt.

23. Verfahren nach einem der vorstehenden Ansprüche, wobei der zweite Katalysator 3 bis 13 Gew.-% fein verteiltes Kupfer, das auf einem Aluminiumoxidträger von hoher Reinheit dispergiert ist, enthält.

24. Verfahren nach einem der vorstehenden Ansprüche, wobei der Aluminiumoxidträger des zweiten Katalysators durch Zersetzen von Triethylaluminium zu alpha-Aluminium-monohydrat, das dann zu gamma-Aluminiumoxid calciniert worden ist, hergestellt worden ist.

## Revendications

1. Procédé pour l'hydrogénation sélective d'alkynes présents dans des coupes $C_4$ riches en 1,3-butadiène, comprenant les étapes suivantes:
   (i) prendre une coupe $C_4$ riche en 1,3-butadiène;
   (ii) passer cette coupe, au moins partiellement à l'état liquide, sur un premier catalyseur à base de palladium en présence d'hydrogène;
   (iii) passer l'effluent provenant de l'étape (ii), au moins partiellement à l'état liquide, sur un second catalyseur à base de cuivre en présence d'hydrogène;
   (iv) séparer l'hydrogène résiduel du reste de l'effluent provenant de l'étape (iii); et
   (v) récupérer une charge riche en 1, 3-butadiène.

2. Procédé selon la revendication 1, caractérisé en ce que la pression totale est comprise entre 0,4 MPa et 0,9 MPa lorsque le courant d'hydrogène utilisé est constitué de 100% d'hydrogène.

3. Procédé selon la revendication 2, caractérisé en ce que la pression totale est comprise entre 0,6 MPa et 0,8 MPa.

4. Procédé selon l'une quelconque des revendications 1 à 3, caractérisé en ce que le rapport molaire hydrogène:alkynes vaut de 2:1 à 20:1.

5. Procédé selon la revendication 4, caractérisé en ce que le rapport molaire hydrogène:alkynes vaut de 4:1 à 10:1.

6. Procédé selon la revendication 5, caractérisé en ce que le rapport molaire hydrogène:alkynes vaut environ 6:1.

7. Procédé selon l'une quelconque des revendications précédentes, caractérisé en ce que jusqu'à 30% du courant total d'hydrogène est injecté en un ou plusieurs endroits le long du premier lit catalytique jusqu'à l'entrée du second réacteur.

8. Procédé selon l'une quelconque des revendications précédentes, caractérisé en ce que la coupe $C_4$ est passée sur le premier catalyseur en phase mixte.

9. Procédé selon l'une quelconque des revendications précédentes, caractérisé en ce que l'étape (ii) est effectuée dans un réacteur adiabatique.

10. Procédé selon l'une quelconque des revendications précédentes, caractérisé en ce qu'une proportion suffisante de la charge est injectée sous forme liquide en un ou plusieurs endroits le long du premier lit catalytique.

11. Procédé selon la revendication 10, caractérisé en ce que jusqu'à environ 20% de la charge est injectée sous forme liquide en un ou plusieurs endroits à environ mi-chemin de la longueur du lit catalytique.

12. Procédé selon l'une quelconque des revendications précédentes, caractérisé en ce que l'étape (ii) est effectuée en mode d'écoulement descendant.

13. Procédé selon l'une quelconque des revendications précédentes, caractérisé en ce que le premier catalyseur contient de 0,1 à 0,35% en poids de palladium métal actif déposé sur une alumine de grande pureté.

14. Procédé selon la revendication 13, caractérisé en ce que la quantité du palladium métal actif dans le premier catalyseur est d'environ 0,2% poids.

15. Procédé selon l'une quelconque des revendications précédentes, caractérisé en ce que le premier catalyseur est stabilisé par l'utilisation d'un catalyseur bimétallique.

16. Procédé selon la revendication 15, caractérisé en ce que le premier catalyseur est constitué d'un alliage or-platine déposé sur une alumine de grande pureté.

17. Procédé selon l'une quelconque des revendications précédentes, caractérisé en ce que la VSHL de la charge dans l'étape (ii) est ajustée de telle manière que la concentration en alkynes après passage sur le catalyseur à base de palladium soit de 300 à 400 ppm poids.

18. Procédé selon l'une quelconque des revendications 1 à 16, caractérisé en ce que la VSHL (exprimée en l/l.h) de la charge dans l'étape (ii) est telle que le produit de cette VSHL par la concentration initiale en alkynes (exprimée en % poids) vaut environ 15.

19. Procédé selon l'une quelconque des revendications précédentes, caractérisé en ce que la température d'entrée de la charge pour l'étape (iii) est comprise entre 45 et 70°C.

20. Procédé selon l'une quelconque des revendications précédentes, caractérisé en ce que la VSHL de la charge à l'étape (iii) est inférieure à environ 6,0 l/l.h.

21. Procédé selon la revendication 20, caractérisé en ce que la VSHL de la charge à l'étape (iii) est d'environ 5,5 1/1.h.

22. Procédé selon l'une quelconque des revendications précédentes, caractérisé en ce que dans l'étape (iii), le rapport molaire hydrogène:alkynes est supérieur à environ 1:5.

23. Procédé selon l'une quelconque des revendications précédentes, caractérisé en ce que le deuxième catalyseur comprend de 3 à 13% en poids de cuire finement divisé dispersé sur un support en oxyde d'aluminium de grande pureté.

24. Procédé selon l'une quelconque des revendications précédentes, caractérisé en ce que le support en oxyde d'aluminium du deuxième catalyseur est préparé par décomposition de triéthyl aluminium en monohydrate d'aluminium alpha qui est ensuite calciné en alumine gamma.